# EUROPÄISCHE PATENTANMELDUNG

(11) **EP 2 492 259 A1**
(43) Veröffentlichungstag der Anmeldung: **29.08.2012**
(21) Anmeldenummer: 11156107.2
(22) Anmeldetag: 25.02.2011
(51) Int. Cl.: C07C 323/62, C07C 323/67, C07D 251/38, C07C 303/08, C07C 319/02, C07C 319/06, C08F 8/34, C08F 14/06, C08K 5/098

(54) **Thiolate als nicht-migrierende PVC-Weichmacher**

(71) Anmelder: ecoatech GmbH, 86167 Augsburg (DE); Consejo Superior De Investigaciones Científicas, 28006 Madrid (ES)
(72) Erfinder: Reinecke, Helmut, E28006, Madrid (ES); Navarro, Rodrigo, E28006, Madrid (ES); Perez, Monica, E28006, Madrid (ES); Gallardo, Alberto, E28006, Madrid (ES); Niederstadt, Rule, 86157, Augsburg (DE)
(74) Vertreter: Reitstötter - Kinzebach

(57) **Zusammenfassung**

Thiolatverbindungen der allgemeinen Formel I werden als Weichmacher für PVC verwendet, 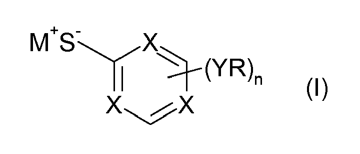 worin
M⁺
für H oder ein Kationäquivalent steht,
n
für 1 oder 2 steht,
X
unabhängig voneinander für eine Gruppe CH oder N stehen,
Y
an ein Ring-C-Atom gebunden ist und unabhängig voneinander für *-CO-O-, *-O-CO-, *-CO-NR'-, *-NR'-CO-, *-SO₂-NR'-, *-NH-CO-O-, *-NH-CO-NR'-, *-O-, *-S- oder *-NR'- steht, wobei * den Anknüpfungspunkt an das Ringsystem beschreibt,
R
für einen Polyalkylenoxid-Rest steht, oder, wenn wenigstens ein X für N steht, R auch für (C₆-C₂₂)-Alkyl stehen kann,
R'
für Wasserstoff, (C₁-C₄)-Alkyl oder R steht.

## Beschreibung

Die Erfindung betrifft nicht-migrierende PVC-Weichmacher auf Basis von Thiolatverbindungen, Verfahren zu deren Herstellung sowie Polyvinylchloride (PVC), die mit diesen Weichmachern kovalent modifiziert wurden.

PVC-Formulierungen finden Einsatz in der Produktion von vielerlei Artikeln im medizinischen Bereich, zum Beispiel in Transfusionsschläuchen und Blut- und Urinbeuteln. Sie werden außerdem als Verpackungsmaterialien eingesetzt, in Spielzeug, Badezimmervorhängen und Fußbodenbelägen. Damit das PVC die erforderliche Flexibilität erhält, werden ihm große Mengen von Weichmachern zugesetzt. Phthalate wie Dioctylphthalat (DOP) gehören zu den am häufigsten verwendeten Additiven für diesen Zweck. Aus thermodynamischen Gründen haben diese Weichmacher die Tendenz, an die Oberfläche des betreffenden Artikels zu migrieren. Dies führt zum Einem zu einem fortschreitenden Verlust der ursprünglichen Eigenschaften des Materials und kann zum Anderen erhebliche Gesundheitsschäden verursachen, wenn es sich um Artikel für biomedizinische Anwendungen oder Kinderspielzeug handelt, bei denen die an die Oberfläche migrierten Additive in den menschlichen Organismus gelangen und physiologische Flüssigkeiten wie Blut, Speichel oder Plasma kontaminieren. So ist gezeigt worden, dass Phthalate menschliches Gewebe schädigen können, wobei besonders die Hirnanhangdrüse (Hypophyse), die Leber und die Hoden betroffen sind. Metabolite von DOP wurden zudem als stark krebsfördernde Substanzen identifiziert. Aus all diesen Gründen wird seit langem daran gearbeitet, die Migration von Weichmachern in die Umwelt zu reduzieren.

Eine vielversprechende Strategie, um das Migrationsproblem zu lösen, ist die kovalente chemische Anbindung des Additivs an die Polymerkette, was zu copolymerartigen Strukturen führt. PVC kann durch Substitution der Chloratome durch geeignete Nukleophile chemisch modifiziert werden.

Die Möglichkeit, substituierte Thiole zu verwenden, um niedermolekulare Weichmacher irreversible an die PVC-Ketten zu binden, ist in der FR 2557115A1 beschrieben. In dieser Arbeit wurde zum Einen 2-Thiosalicylsäure(2-ethyl-hexyl)ester verwendet und zum Anderen α-Thioglycolsäure (2-ethyl-hexyl)ester. Beide Substanzen konnten nur in begrenztem Ausmaß an die Polymerkette gebunden werden, wobei weder in Lösung noch in der Schmelze Umsätze von über 30 mol% erreicht wurden. Entsprechend war auch die erzielte Weichmacherwirkung beschränkt und Glastemperaturen von 25°C wurden nicht unterschritten. Heutzutage ist bekannt, dass die Ursachen der Begrenzung der erreichten Umsätze als auch die der Glastemperaturen in der Struktur der gewählten Thiolverbindungen liegen. Zum Einen sind aliphatische Thiole nicht ausreichend nukleophil, um die Chloratome in PVC zu substituieren, zum Anderen sind ortho-substituierte Thiole sterisch gehindert, was zu geringen Umsätzen führt. Des Weiteren führt die Verwendung von ortho-substituierten Thiolen aufgrund sterischer Effekte zu einer Versteifung der betreffenden Kettensegmente was sich wiederum negativ auf den gewünschten Weichmachereffekt des eingebrachten Moleküls auswirkt.

In der WO 2010/070176A1 wird die Anbindung von Thiol-modifiziertem Dioctylphtalat an PVC beschrieben. Auch hier sind die erzielten Umsätze auf 25 mol% beschränkt. Aufgrund der sterisch günstigeren Anordnung der aliphatischen Estergruppen konnten hier Glasübergangstemperaturen um 0°C für die am höchsten modifizierten Polymere erzielt werden. Um einen ausreichenden Weichmachereffekt zu erzielen, muss jedoch ein hoher Anteil der Chloratome - bis zu einem Viertel aller Chloratome im PVC - substituiert werden. Hierdurch kann es zu einer Verschlechterung der optischen Eigenschaften, z. B. durch Vergilbung, und der mechanischen Eigenschaften kommen. Ein weiterer Nachteil, der den Gebrauch der in dieser Anmeldung beschriebenen Technologie für einen industriellen Nutzen erheblich einschränkt, ist auch hier die Notwendigkeit, das erhaltene Produkt von nicht umgesetztem Weichmacher zu befreien.

Die Aufgabe der vorliegenden Erfindung liegt in der Bereitstellung von nicht-migrierenden PVC-Weichmachern, die bereits bei einem geringen PVC-Substitutionsgrad einen ausreichenden Weichmachereffekt zeigen. Glastemperaturen und mechanische Eigenschaften des modifizierten PVCs sollen mit denen eines PVCs vergleichbar sein, das mit konventionellen, migrierenden Weichmachern behandelt wurde. Die Modifizierung des PVCs mit dem nicht-migrierenden Weichmacher soll dabei so vollständig ablaufen, dass eine Aufreinigung des PVCs überflüssig ist.

Diese und weitere Aufgaben werden überraschender Weise gelöst durch Weichmacher auf Basis aromatischer Thiolatverbindungen, die Polyalkylenoxidreste tragen.

Ein erster Gegenstand der Erfindung ist daher eine Thiolatverbindung der allgemeinen Formel I 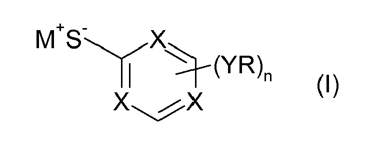 worin
- M⁺: für H oder ein Kationäquivalent steht,
- n: für 1 oder 2 steht,
- X: unabhängig voneinander für eine Gruppe CH oder N stehen,
- Y: an ein Ring-C-Atom gebunden ist und unabhängig voneinander für *-CO-O-, *-O-CO-, *-CO-NR'-, *-NR'-CO-, *-SO₂-NR'-, *-NH-CO-O-, *-NH-CO-NR'-, *-O-, *-S-oder *-NR'- steht, wobei * den Anknüpfungspunkt an das Ringsystem beschreibt,
- R: für einen Polyalkylenoxid-Rest steht, oder, wenn wenigstens ein X für N steht, R auch für (C₆-C₂₂)-Alkyl stehen kann,
- R': für Wasserstoff, (C₁-C₄)-Alkyl oder R steht.

Ein weiterer Gegenstand der Erfindung ist modifiziertes PVC, das Wiederholungseinheiten der Formeln II.a und II.b enthält, 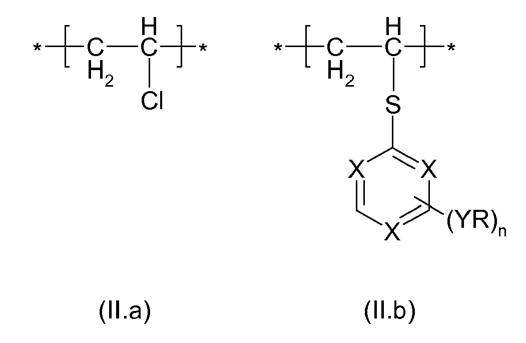 worin n, R, X und Y für zuvor genannten Bedeutungen haben.

Das molare Verhältnis der Wiederholungseinheiten der Formel II.b zu den Wiederholungseinheiten der Formel II.a beträgt vorzugsweise 1:200 bis 1:9, insbesondere 1:100 bis 1:30.

Die Erfindung betrifft außerdem die Verwendung des modifizierten PVCs in biomedizinischen Anwendungen, in Kinderspielzeug und in Anwendungen, in denen das modifizierte PVC in Kontakt mit dem menschlichen Organismus oder physiologischen Flüssigkeiten tritt.

Der Ausdruck "Kationäquivalent" steht in der vorliegenden Anmeldung für ein einfach positiv geladenes Kation oder den fiktiven, einfach positiv geladenen Bruchteil eines mehrfach positiv geladenen Kations.

In der Formel I steht M⁺ für H oder ein Kationäquivalent, vorzugsweise ein Kationäquivalent, insbesondere ein Ammoniumion, Alkalimetall-Kation oder ein Kationäquivalent eines Erdalkalimetall-Kations. Vorzugsweise steht M⁺ für ein Alkalimetall-Kation wie Na⁺ oder K⁺.

Die Variable "n" steht für 1 oder 2.

Die Variable "X" steht unabhängig für N oder CH. Vorzugsweise steht X ausschließlich für N oder ausschließlich für CH. In einer bevorzugten Ausführungsform steht X ausschließlich für CH und n für 1. In einer weiteren bevorzugten Ausführungsform steht X ausschließlich für N und n für 2.

In der allgemeinen Formel I steht R für einen Polyalkylenoxid-Rest. Unter einem Polyalkylenoxid-Rest wird ein Rest verstanden, der Alkylenoxid-Wiederholungseinheiten umfasst. Die Alkylenoxid-Wiederholungseinheiten leiten sich vorzugsweise von (C₂-C₆)-Alkylenoxiden ab. In bestimmten Ausführungsformen ist der Polyalkylenoxid-Rest am distalen Ende verethert.

Bevorzugte Reste R weisen die allgemeine Formel auf:

-Qₖ-(CₓH₂ₓ-O)ₘ-R"

worin
- k: für 0 oder 1 steht,
- x: für eine ganze Zahl von 2 bis 6 steht,
- m: für 3 bis 1000 steht,
- Q: für C₂-C₂₀-Alkylen, vorzugsweise C₂-C₈-Alkylen, steht und
- R": für Wasserstoff C₁-C₂₄-Alkyl, vorzugsweise Wasserstoff oder C₁-C₂₄-Alkyl, steht.

Bevorzugte Polyalkylenoxide sind Poly((C₂-C₆)-alkylenoxide) wie Poly(ethylenoxid) oder Poly(propylenoxid) oder Copolymere verschiedener (C₂-C₆)-Alkylenoxide, wie Copolymere aus Ethylenoxid und Propylenoxid.

Wenn wenigstens ein X für N steht, kann R auch für (C₆-C₂₂)-Alkyl, vorzugsweise (C₈-C₁₈)-Alkyl stehen. Der Alkylrest kann geradkettig oder verzweigt sein und ist vorzugsweise verzweigt.

Der Substituent R' steht für Wasserstoff oder (C₁-C₄)-Alkyl oder hat die für R genannten Bedeutung. Bevorzugt steht R' für Wasserstoff oder (C₁-C₄)-Alkyl, insbesondere für Wasserstoff oder Methyl.

In der allgemeinen Formel I ist Y an ein Ring-C-Atom gebunden und steht unabhängig voneinander für *-CO-O-, *-O-CO-, *-CO-NR'-, *-NR'-CO-, *-SO₂-NR'-, *-NH-CO-O-, *-NH-CO-NR'-, *-O-, *-S- oder *-NR'-. Bevorzugt steht Y für *-CO-O-, *-CO-NR'-, *-SO₂-NR'-, *-NH-CO-O- oder *-O-. Insbesondere steht Y für *-CO-NR'-, *-SO₂-NR'-, *-NH-CO-O- oder *-O-.

Die Thiolatverbindung der allgemeinen Formel I weist ein Molekulargewicht von mindestens 400 g/mol, vorzugsweise 600 bis 10000 g/mol, insbesondere 800 bis 2000 g/mol auf.

Das erfindungsgemäß modifizierte PVC weist hervorragende mechanische und optische Eigenschaften auf. Es ist transparent, flexibel, dauerelastisch und zeigt eine hohe Reißfestigkeit. Weiterhin ist die Glasübergangstemperatur des modifizierten PVCs im Vergleich zu nicht modifiziertem PVC erniedrigt. Schon ein geringer Anteil an Wiederholungseinheiten der Formel llb im modifizierten PVC führt zu den beschriebenen verbesserten mechanischen Eigenschaften.

Der kovalent gebundene erfindungsgemäße Weichmacher migriert nicht. Aufgrund der damit verbundenen verringerten Toxizität kann das erfindungsgemäß modifizierte PVC auch in Anwendungen eingesetzt werden, in denen Menschen in direkten Kontakt mit dem modifizierten PVC kommen. Folglich ist ein weiterer Aspekt der vorliegenden Erfindung die Verwendung des modifizierten PVCs in biomedizinischen Anwendungen, in Kinderspielzeug und in Anwendungen, in denen das modifizierte PVC in Kontakt mit dem menschlichen Organismus oder physiologischen Flüssigkeiten tritt.

Das PVC kann durch Emulsions-, Suspensions- oder Masse-Polymerisation hergestellt sein.

Zur Modifizierung wird PVC mit der Thiolatverbindung gemäß Formel I umgesetzt, in der M⁺ für ein Kationäquivalent steht, oder in Gegenwart einer Base mit der Thiolatverbindung gemäß Formel I umgesetzt, worin M⁺ für H steht. Eine geeignete Base ist Natriumcarbonat. Die Modifizierung kann lösungsmittelfrei oder in Wasser suspendiert oder in organischen Lösungsmitteln gelöst erfolgen. Bevorzugt wird das PVC in Wasser suspendiert oder lösungsmittelfrei umgesetzt. Als organisches Lösungsmittel werden dem Fachmann bekannte Lösungsmittel für PVC wie Tetrahydrofuran oder Dimethylformamid verwendet. Die lösungsmittelfreie Einarbeitung des erfindungsgemäßen Weichmachers in das PVC erfolgt z. B. auf einem Mischwalzwerk, z.B. einem Zweiwalzenstuhl bei Temperaturen zwischen 150° und 200°C. Alternativ kann Suspensions-PVC in Form einer wässrigen Paste mit dem erfindungsgemäßen Weichmacher bei Temperaturen zwischen 40° und 90°C umgesetzt werden. Unter den Bedingungen der Einarbeitung erfolgt eine nukleophile Substitution von Chloratomen des PVC durch die Thiolatgruppe des erfindungsgemäßen Weichmachers.

In einer bevorzugten Ausführungsform entspricht die erfindungsgemäße Thiolatverbindung der allgemeinen Formel I.a 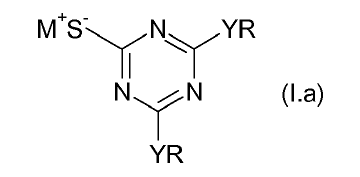 worin M⁺, Y und R die oben angegebene Bedeutung haben.

In der Formel I.a steht Y vorzugsweise für *-O-, *-S- oder *-NR'-. In der Formel I.a steht R vorzugsweise für für (C₆-C₂₂)-Alkyl, insbesondere für (C₈-C₁₈)-Alkyl.

In einer weiteren bevorzugten Ausführungsform entspricht die erfindungsgemäße Thiolatverbindung der allgemeinen Formel I.b 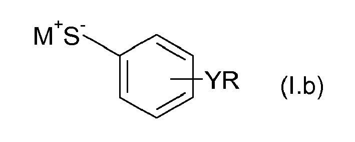 worin M⁺, Y und R die oben angegebene Bedeutung haben.

In der Formel I.a steht Y vorzugsweise für *-CO-O-, *-O-CO-, *-CO-NR'-, *-SO₂-NR'-, *-NH-CO-O-, *-NH-CO-NR'- oder *-O-.

Ein weiterer Aspekt der vorliegenden Erfindung sind Verfahren zur Herstellung von Thiolatverbindungen gemäß der allgemeinen Formel I. Diese Verbindungen können insbesondere durch die folgenden Verfahren hergestellt werden.

In einem bevorzugten Verfahren wird eine Thiolatverbindung der allgemeinen Formel I.b hergestellt, worin Y für *-CO-O-, *-CO-NR'-, *-NH-CO-O-, *-NH-CO-NR'- steht, indem man
1) ein Disulfid der Formel III.a 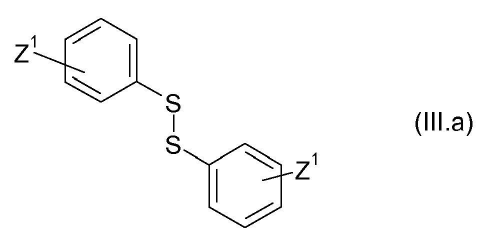 worin Z¹ für CO-Z³ oder NCO steht, wobei Z³ für Halogen oder Acyloxy steht,
   mit einer Verbindung der Formel R-Z²H, worin Z² für O oder NR' steht, zum Disulfid der Formel III.b umsetzt,
   und 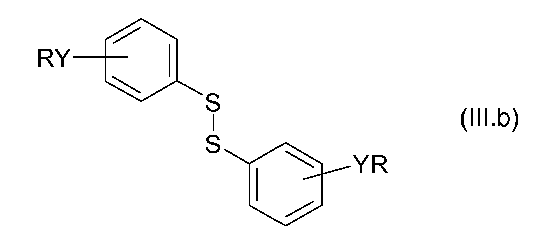
2) das Disulfid der Formel III.b in eine Thiolatverbindung der Formel I.b umwandelt. Die im Schritt 1) eingesetzten Disulfide lassen sich leicht durch oxidative Dimerisierung von Thiolverbindungen der Formel IV.a, 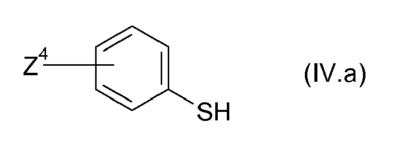
worin Z⁴ für Z¹ oder eine Gruppe steht, die in Z¹ umgewandelt werden kann, und gegebenenfalls Umwandlung von Z⁴ in Z¹ erhalten.

Die Thiolverbindungen IV.a sind handelsüblich oder lassen sich durch dem Fachmann bekannte Verfahren herstellen.

Die Dimerisierung der Thiolverbindung in Schritt 1 zu einem Disulfid erfolgt durch Oxidation. Entsprechende Oxidationsmittel zur Herstellung eines Disulfides sind dem Fachmann bekannt. Bevorzugt werden H₂O₂ und Persäuren mit 1 bis 10 Kohlenstoffatomen, insbesondere H₂O₂ eingesetzt.

Z⁴ steht beispielsweise für COOH oder NH₂. Die Gruppe COOH kann durch übliche Halogenierungsmittel, wie Thionylchlorid (SOCl₂), Phosgen (COCl₂), Phosphor(III)-chlorid oder Phosphor(V)-chlorid, in CO-Z³ umgewandelt werden, worin Z³ für Halogen steht. Die Gruppe COOH kann durch übliche Acylierungsmittel, wie Essigsäureanhydrid oder dem gemischten Anhydrid von Ameisensäure und Essigsäure, in CO-Z³ umgewandelt werden, worin Z³ für Acyloxy steht.

Die Gruppe NH₂ kann z. B. durch Umsetzung mit Phosgen in eine Isocyanatgruppe (NCO) umgewandelt werden.

Als Verbindungen der Formel R-Z²H sind handelsübliche Polyoxyalkylenamine verwendbar, die unter der Bezeichnung Jeffamine® von Huntsman erhältlich sind. Als Verbindungen der Formel R-Z²H sind weiterhin Polyoxyalkylenalkohole verwendbar.

Die Umsetzung des Disulfids III.a, worin Z¹ für CO-Z³ steht, erfolgt vorzugsweise in Gegenwart einer Base, wie Kaliumcarbonat oder Triethylamin.

Die Umwandlung des Disulfides der Formel III.b in eine Thiolatverbindung der Formel I.b in Schritt 2) erfolgt bevorzugt durch reduktive Spaltung der Disulfidbrücke. Reduktionsmittel zur reduktiven Spaltung von Disulidbrücken sind dem Fachmann bekannt. Bevorzugt werden Aluminiumhydride oder Borhydride, insbesondere Alkali-Aluminiumhydride oder Alkali-Borhydride, speziell Natriumborhydrid oder Kaliumborhydrid eingesetzt. Diese führen unmittelbar zum Alkalisalz des Thiolats der Formel I.b. Bei Verwendung anderer Reduktionsmittel ist gegebenenfalls eine anschließende Neutralisation mit einem geeigneten Neutralisationsmittel erforderlich.

Ein Austausch des Gegenions des Thiolates wird gewünschtenfalls durch Protonierung des Thiolates mit einer Säure und anschließender Deprotonierung des gebildeten Thiols mit einer Base erreicht. Bevorzugt werden als Säuren ein- bis drei-protonige anorganische Säure wie Salzsäure, Schwefelsäure oder Phosphorsäure eingesetzt. Als Base werden bevorzugt Alkali- und Erdalkalibasen wie Na₂CO₃, K₂CO₃, NaOH, KOH bzw. CaCO₃ oder Stickstoffbasen wie Ammoniak eingesetzt.

In einem bevorzugten Verfahren wird eine Thiolatverbindung der allgemeinen Formel I.b hergestellt, worin Y für *-O-CO-, *-NR'-CO-, *-O- oder *-NR'- steht, indem man
1) ein Disulfid der Formel III.c 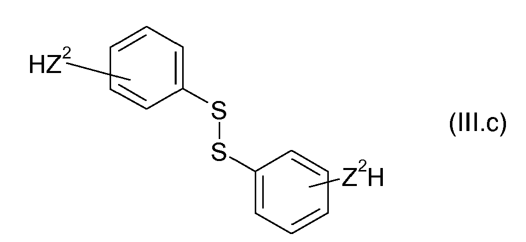 worin Z² für O oder NR' steht, mit einer Verbindung der Formel R-Z⁵, worin Z⁵ für CO-Z³ oder eine Abgangsgruppe steht, wobei Z³ für Halogen oder Acyloxy steht, zum Disulfid der Formel III.d umsetzt, und 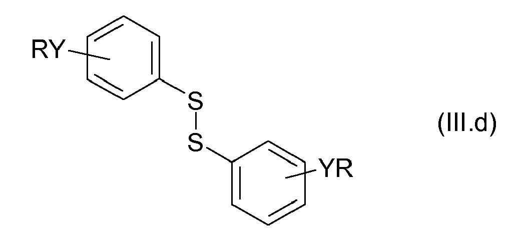
2) das Disulfid der Formel III.d in eine Thiolatverbindung der Formel I.b umwandelt.

Die im Schritt 1) eingesetzten Disulfide lassen sich leicht durch oxidative Dimerisierung von Thiolverbindungen der Formel IV.b, 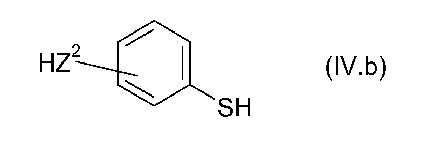 herstellen. Die Thiolverbindungen IV.b sind handelsüblich, wie z. B. Mercaptophenole oder Mercaptoanilin. Zu den Bedingungen der oxidativen Dimerisierung wird auf die vorstehenden Ausführungen verwiesen.

Geeignete Abgangsgruppen Z⁵ sind z. B. Halogen, wie Chlor, Brom, lod; Methylsulfat, Tosylat, Methylsulfonat, Trifluormethansulfonat.

Verbindungen der Formel R-Z⁵, worin Z⁵ für CO-Z³ steht, sind erhältlich durch dem Fachmann bekannte Verfahren.

Verbindungen der Formel R-Z⁵, worin Z⁵ für eine Abgangsgruppe steht, sind beispielsweise erhältlich, indem man die Alkoholfunktion in einem Polyoxyalkylenalkohole in eine Abgangsgruppe umwandelt, z. B. durch Halogenierung oder Veresterung mit Toluolsulfonsäure oder dergleichen.

Die Umsetzung des Disulfids der Formel III.c mit der Verbindung der Formel R-Z⁵ erfolgt vorzugsweise in Gegenwart einer Base, wie Kaliumcarbonat oder Triethylamin.

Die Umwandlung des Disulfides der Formel III.d in eine Thiolatverbindung der Formel I.b in Schritt 2) erfolgt bevorzugt durch reduktive Spaltung der Disulfidbrücke. Hierzu wird auf die vorstehenden Ausführungen verwiesen.

In einem bevorzugten Verfahren wird eine Thiolatverbindung der allgemeinen Formel I.b hergestellt, worin Y für *-SO₂-NR'- steht, indem man
1) ein Disulfid der Formel III.e 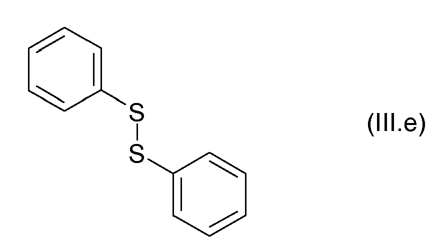 zum Sulfonylchlorid der Formel III.f chlorsulfoniert, 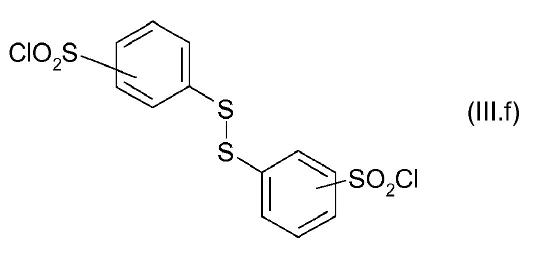
2) das Sulfonylchlorid der Formel III.f mit einer Verbindung der Formel R-Z²H, worin Z² für O oder NR' steht, zum Disulfid der Formel III.g umsetzt, 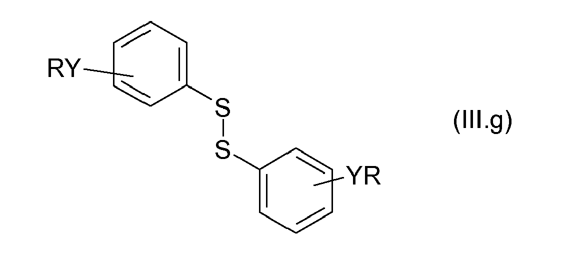
3) das Disulfid der Formel I.e in eine Thiolatverbindung der Formel I.b umwandelt.

Die Chlorsulfonierung in Schritt 1) erfolgt in üblicher Weise z. B. mittels Chlorsulfonsäure.

Geeignete Verbindungen der Formel R-Z²H sind die weiter oben genannten.

Die Umsetzung des Sulfonylchlorids mit der Verbindung der Formel R-Z²H erfolgt vorzugsweise in Gegenwart einer Base, wie Kaliumcarbonat oder Triethylamin.

Die Umwandlung des Disulfides der Formel I.e in eine Thiolatverbindung der Formel I in Schritt 3) erfolgt bevorzugt durch reduktive Spaltung der Disulfidbrücke. Hierzu wird auf die vorstehenden Ausführungen verwiesen.

In einem bevorzugten Verfahren wird eine Thiolatverbindung der allgemeinen Formel I.b hergestellt, worin Y für *-O- steht, indem man
1) ein Disulfid der Formel III.h 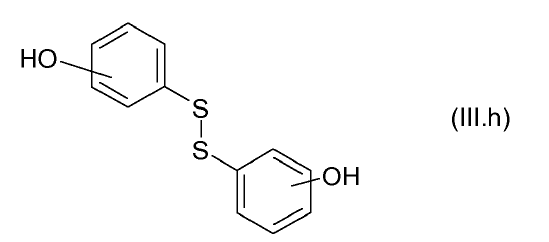 in Gegenwart einer Base mit einem Alkylenoxid der Formel CₓH₂ₓO, worin x für eine ganze Zahl von 2 bis 6 steht, zu einem Disulfid der Formel III.i umsetzt, worin m für 3 bis 100 steht, und 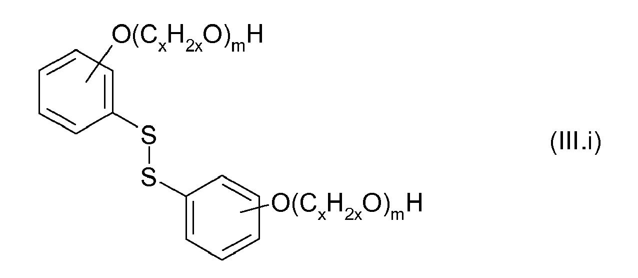
2) das Disulfid der Formel III.i in eine Thiolatverbindung der Formel I.b umwandelt.

Die im Schritt 1) eingesetzten Disulfide lassen sich leicht durch oxidative Dimerisierung von Mercaptophenol erhalten.

Bevorzugte Alkylenoxide sind Ethylenoxid, Propylenoxid oder 1,2-Butylenoxid.

Eine geeignete Base ist Natriumhydrid.

Die Umwandlung des Disulfides der Formel III.i in eine Thiolatverbindung der Formel I in Schritt 3) erfolgt bevorzugt durch reduktive Spaltung der Disulfidbrücke. Hierzu wird auf die vorstehenden Ausführungen verwiesen.

Ebenfalls bevorzugt ist ein Verfahren zur Herstellung der Thiolatverbindung der Formel I.b, bei dem man
1) eine Verbindung der Formel IV.c, 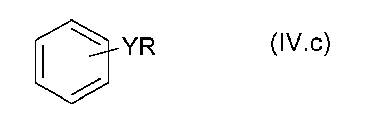 chlorsulfoniert, und
2) das erhaltene Sulfonylchlorid der Formel IV.d 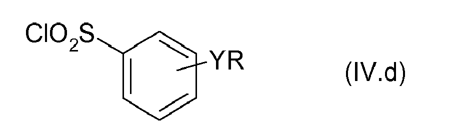 in eine Thiolatverbindung der Formel I.b umwandelt.

Die in Schritt 1 als Edukt eingesetzte Verbindung der Formel IV.c lässt sich nach Verfahren herstellen, die einem Fachmann bekannt sind. So kann man z. B. Benzoesäurechlorid in Gegenwart einer Base mit einer Verbindung der Formel R-Z²H umsetzen, worin Z² für O oder NR' steht.

Die Chlorsulfonierung in Schritt 1 wird bevorzugt durchgeführt, indem die Verbindung gemäß Formel IV.c mit Chlorsulfonsäure umgesetzt oder mit bekannten Sulfonierungsmitteln wie Oleum oder Schwefeltrioxid sulfoniert und anschließend die eingeführte Sulfonylgruppe mit bekannten Chlorierungsreagenzien wie PCl₃, PCl₅, Phosgen und Oxalylchlorid chloriert wird.

In Schritt 2 wird das Sulfonylchlorid der Formel IV.d durch Reduktion in eine Thiolatverbindung der Formel I.b überführt. Dabei wird das Sulfonylchlorid mit dem Fachmann bekannten Reduktionsmitteln für Sulfonylchloriden wie beispielsweise SnCl₂/HCl reduziert und anschließend die entstehende Thiolverbindung durch Umsetzung mit Neutralisationsmittel in eine Thiolatverbindung der Formel I.b überführt. Als Neutralisationsmittel werden bevorzugt Alkali- und Erdalkalibasen wie Na₂CO₃, K₂CO₃, NaOH, KOH bzw. CaCO₃ oder Stickstoffbasen wie Ammoniak eingesetzt.

Weiterhin ist ein Verfahren zur Herstellung einer Thiolatverbindung der Formel I.a bevorzugt, worin Y für *-O-, *-S- oder *-NR'- steht,
wobei man
1) ein [1,3,5]-Triazin der Formel V.a 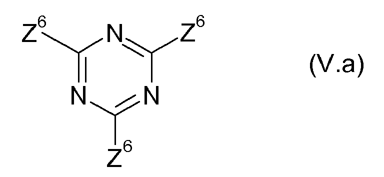 worin Z⁶ für eine Abgangsgruppe steht
   mit wenigstens einer Verbindung der Formel R-Z²H, worin Z² für O oder NR' steht, zu einer Verbindung der Formel V.b umsetzt, 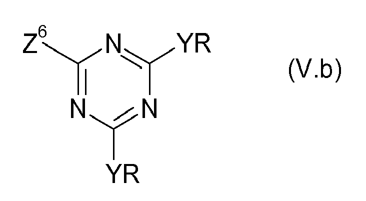
2) die Verbindung der Formel V.b mit Thioharnstoff zu einem Thiuroniumsalz der Formel V.c umsetzt 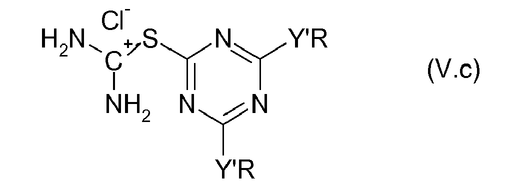 und
3) das erhaltene Thiouroniumsalz durch Umsetzung mit einer Base in die Thiolatverbindung der Formel I.a umwandelt.

Geeignete Abgangsgruppen Z⁶ sind z. B. Halogen, wie insbesondere Chlor.

Das in Schritt 1 eingesetzte [1,3,5]-Triazin der Formel V.a ist handelsüblich. Ein bevorzugtes [1,3,5]-Triazin der Formel I.h ist 2,4,6-Trichlor-[1,3,5]-triazin.

Geeignete Verbindungen der Formel R-Z²H sind die weiter oben genannten.

Die Umsetzung der Verbindung der Formel V.b mit Thioharnstoff zu dem ThiuroniumSalz der Formel V.c in Schritt 2 erfolgt vorzugsweise in einem geeigneten Lösungsmittel bei Temperaturen in einem Bereich von -50 bis 70 °C, vorzugsweise -20 bis 40 °C, insbesondere -10 bis 20 °C. Als organisches Lösungsmittel werden Alkanole bevorzugt, insbesondere C₁-C₆-Alkanole wie Methanol, Ethanol, 1-Propanol oder 2-Propanol. Thioharnstoff und die Verbindung V.b werden regelmäßig in einem molaren Verhältnis in einem Bereich von 1:10 bis 10:1, bevorzugt von 2:1 bis 1:4, insbesondere von 1:1 bis 3:1, eingesetzt.

In Schritt 3 wird das Thiuroniumsalz der Formel V.c bevorzugt mit einer Alkali-Base wie Na₂CO₃, K₂CO₃, NaOH oder KOH umgesetzt. Bevorzugt wird dabei die Base im Überschuss eingesetzt.

Die beigefügte Figur und die nachfolgenden Beispiele sollen die Erfindung weiter erläutern und sind nicht einschränkend zu verstehen.

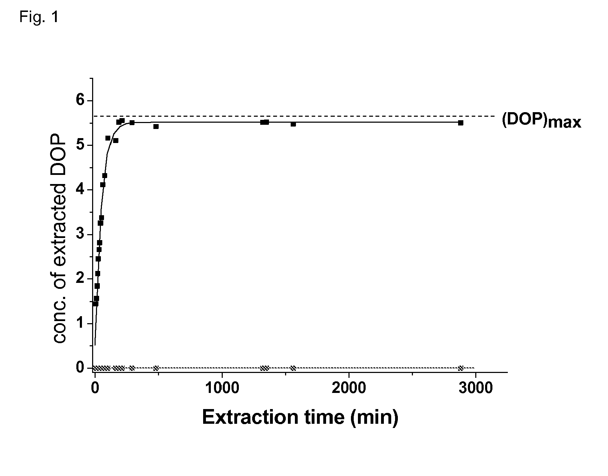

Fig. 1 zeigt die Ergebnisse von Extraktionsversuchen, durchgeführt an konventionell weichgemachtem PVC (obere Kurve) und an PVC mit erfindungsgemäß weichgemachten (untere Kurve)

### 1. Herstellung der Thiolatverbindungen der Formel I

### 1.1 Herstellung von Polypropylenoxy-4-mercaptobenzamid

### 1.1.1 4,4'-Disulfandiyldibenzoesäure

10 g (0,065 mol) 4-Mercaptobenzoesäure werden in einer Mischung aus einem Teil destilliertem Wasser und einem Teil Methanol (v/v) gelöst und unter Rühren werden 2 ml H₂O₂ (30%) und 0,5 ml Ammoniakwasser zugegeben. Nach weiteren 30 Minuten Rühren bei Raumtemperatur wird der gebildete Niederschlag abfiltriert. Nach dem Trocknen erhält man ein weißes Pulver, dessen Reinheit ausreichend ist für die nachfolgende Umsetzung. Ausbeute: 90%

### 1.1.2 4,4'-Disulfandiyldibenzoylchlorid

10 g (0,032 mol) 4,4'-Disulfandiyldibenzoesäure werden in 50 ml Thionylchlorid suspendiert. Anschließend werden 0,5 ml Dimethylformamid (DMF) zugegeben und die Mischung 2 Stunden unter Rückfluss erhitzt. Danach wird das überschüssige Thionylchlorid destillativ abgetrennt und der Rückstand in Hexan ausgefällt. Der Niederschlag wird mehrmals mit Hexan gewaschen und getrocknet. Ausbeute: 90%

### 1.1.3 4,4'-Disulfandiyl-bis-(N-polypropylenoxy)benzamid

5 g (0,015 mol) 4,4'-Disulfandiyldibenzoylchlorid werden in 100 ml Chloroform gelöst und bei 0 °C werden stöchiometrischen Mengen an O-(2-Aminopropyl)-O'-(2-methoxyethyl)polypropylenglycol (Jeffamine -2005, Huntsman) und Triethylamin zugegeben. Nach 2 Stunden Rühren bei Raumtemperatur wird die Reaktionslösung dreimal mit destilliertem Wasser gewaschen, die organische Phase mit Magnesiumsulfat getrocknet und das Lösungsmittel am Rotationsverdampfer entfernt. Der erhaltene Rückstand kann ohne weitere Reinigung für den nächsten Reaktionsschritt verwendet werden. Ausbeute: 99%.

### 1.1.4 Polypropylenoxy-4-mercaptobenzamid

10 g (2,3 mmol) 4,4'-Disulfandiyl-bis (N-polypropylenoxy)benzamid werden in 100 ml Isopropanol gelöst und 4 Moläquivalente NaBH₄ werden anschließend hinzugegeben. Nach 12 Stunden Rühren bei Raumtemperatur wird konzentrierte HCl zu der Mischung, bis diese sauer reagiert. Nach zweimaliger Extraktion mit jeweils 150 ml Chloroform, wird die organische Phase dreimal mit einer gesättigten, wässrigen Natriumhydrogencarbonat-Lösung gewaschen, mit Magnesiumsulfat getrocknet und das Lösungsmittel destillativ entfernt. Das erhaltene Öl kann direkt für die Umsetzung mit PVC weiterverwendet werden. Ausbeute: 99%

### 1.2 Herstellung von Polypropylenoxy-4-mercaptobenzolsulfonamid

### 1.2.1 Diphenyldisulfan

10 g (0,09 mol) Thiophenol werden in einer Mischung aus einem Teil destilliertem Wasser und einem Teil Methanol (v/v) gelöst und anschließend werden unter Rühren 2 ml H₂O₂ (30%) und 0,5 ml Ammoniakwasser zugegeben. Nach 30 Minuten Rühren bei Raumtemperatur wird der gebildete Niederschlag abfiltriert. Nach dem Trocknen erhält man ein weißes Pulver, dessen Reinheit ausreichend ist für die nachfolgende Umsetzung. Ausbeute: 93%

### 1.2.2 4,4'-Disulfandiyldibenzensulfonylchlorid

Zu einer Lösung von 0,3 mol Chlorsulfonsäure in Chloroform wird langsam eine Lösung von 0,1 mol Diphenyldisulfan in 100 ml Chloroform zugegeben, ohne dass die Reaktionstemperatur 10°C übersteigt. Nach erfolgter Zugabe wird die Reaktionslösung noch eine Stunde bei 40°C gerührt. Die Mischung wird auf Eis gegossen und das Produkt mit Chloroform extrahiert. Nachdem die organische Phase dreimal einer gesättigten, wässrigen Natriumhydrogencarbonat-Lösung gewaschen und dreimal mit destilliertem Wasser gewaschen wurde, wird die organische Phase mit Magnesiumsulfat getrocknet und das Lösungsmittel destillativ entfernt. Man erhält das Produkt in Form von hellgelben Nadeln. Ausbeute: 70%.

### 1.2.3 4,4'-Disulfandiyl-bis-(N-polypropylenoxy)benzolsulfonamid

5 g (0,012 mol) 4,4'-Disulfandiyldibenzenesulfonylchlorid werden in 80 ml Chloroform gelöst und anschließend wird bei 0°C stöchiometrische Mengen an O-(2-Aminopropyl)-O'-(2-methoxyethyl)polypropylenglycol (Jeffamine M-2005, Huntsman) und Triethylamin zugegeben. Nach 2 Stunden Rühren bei Raumtemperatur wird die Reaktionslösung dreimal mit destilliertem Wasser gewaschen, mit Magnesiumsulfat getrocknet und das Lösungsmittel am Rotationsverdampfer entfernt. Der erhaltene Rückstand kann ohne weitere Reinigung für den nächsten Reaktionsschritt verwendet werden. Ausbeute: 98%.

### 1.2.4 Polypropylenoxy-4-mercaptobenzolsulfonamid

10 g (2,25 mmol) 4,4'-Disulfandiyl-bis-(N-polypropylenoxy)benzolsulfonamid werden in 100 ml Isopropanol gelöst und anschließend werden 4 Moläquivalente NaBH₄ zugegeben. Nach 12 h Rühren bei Raumtemperatur wird konzentrierte HCl zu der Mischung, bis diese sauer reagiert. Nach zweimaliger Extraktion mit jeweils 150 ml Chloroform, wird die organische Phase dreimal mit einer gesättigten, wässrigen Natriumhydrogencarbonat-Lösung gewaschen, mit Magnesiumsulfat getrocknet und das Lösungsmittel destillativ entfernt. Das erhaltene Öl kann direkt für die Umsetzung mit PVC weiterverwendet werden. Ausbeute: 99%

### 1.3 3,5-Di(2-ethyl-hexyl)oxy-2,4,6-triazin-1-Natriumthiolat

### 1.3.1 1,3-Di(2-ethyl-hexyl)oxy-5-chlor-2,4,6-triazin

0,3 mol Cyanurchlorid (1,3,5-Trichlor-2,4,6-triazin) werden in 300 ml Methyl-ethylketon (MEK) gelöst. Anschließend werden bei 0°C 0,1 mol 2-Ethyl-1-hexanol und 0,1 mol Na₂CO₃ zugegeben. Die Reaktionslösung wird auf Raumtemperatur erwärmt und rührt bei dieser Temperatur 30 Minuten gerührt. Dann werden 0,1 mol 2-Ethyl-1-hexanol und 0,1 mol Na₂CO₃ zugegeben. Die Reaktionslösung wird 1 Stunde bei 45°C gerührt und anschließend auf Raumtemperatur abgekühlt. Danach wird die Mischung mit destilliertem Wasser gewaschen, die organische Phase mit Magnesiumsulfat getrocknet und das Lösungsmittel am Rotationsverdampfer entfernt. Das erhaltene Rohprodukt wird im Vakuum destilliert. Ausbeute: ca. 75%.

### 1.3.2 5-Thiuroniumsalz des 1,3-Di(2-ethyl-hexyl)oxy-5-chlor-2,4,6-triazin

0,1 mol 1,3-Di(2-ethyl-hexyl)oxy-5-chlor-2,4,6-triazin werden in 50 ml Ethanol gelöst und bei 0°C werden 0,11 mol Thioharnstoff zugegeben. Nach zwei Stunden Rühren bei Raumtemperatur wird der Ethanol destillativ entfernt und der Rückstand durch Säulenchromatographie gereinigt. Ausbeute: 70%

### 1.3.3 3,5-Di(2-ethyl-hexyl)oxy-2,4,6-triazin-1-Natriumthiolat

0,1 mol des unter 1.3.2 beschriebenen Thiuroniumsalzes werden in einer Mischung aus Ethanol-Wasser 2:1 (v:v) gelöst und anschießend werden 0,11 mol Na₂CO₃ zugegeben. Nach 2 Stunden Rühren bei 40°C wird die Reaktionslösung dreimal mit Chloroform extrahiert, die organische Phase mit Magnesiumsulfat getrocknet und das Lösungsmittel destillativ entfernt. Das erhaltene Öl kann ohne weitere Aufreinigung für die Umsetzungsreaktion mit PVC weiterverwendet werden. Ausbeute: 95%

### 2. Herstellung von modifiziertem PVC

### a) in Lösung

1 g (0.016 mol) PVC werden in 100 ml Cyclohexanon gelöst. Dann gibt man 1g (0.0005 mol) Polypropylenoxy-4-mercaptobenzamid dazu und 1g (0.0094 mol) Na₂CO₃ und erwaermt 16 Stunden unter N₂-Schutzgas und Rühren auf 60°C. Man fällt dann die Mischung in H₂O/MeOH 1:3 aus, saugt das Polymer ab und reinigt es durch dreimaliges Umfällen aus THF(Lösungsmittel)/MeOH (Fällungsmittel). Nach dem Trocknen erhäelt man das modifizierte PVC in einer Ausbeute von 99%.

### b) in wässriger Suspension

1 g (0.016 mol) PVC werden in 100 ml H₂O suspendiert. Dann gibt man 1g (0.0005 mol) Polypropylenoxy-4-mercaptobenzamid dazu und 1g (0.0094 mol) Na₂CO₃ und erwaermt 16 Stunden unter N₂-Schutzgas und Rühren auf 60°C. Dabei bildet sich eine polymerartige Masse, die auf dem Wasser schwimmt und leicht abgetrennt werden kann. Man reinigt sie durch dreimaliges Umfällen aus THF(Lösungsmittel)/MeOH (Fällungsmittel). Nach dem Trocknen erhält man das modifizierte PVC in einer Ausbeute von 95%.

### c) in Substanz

Man mischt in der Kälte 1 g (0.016 mol) Emulsions-PVC mit 1 g (0.0005 mol) Polypropylenoxy-4-mercaptobenzoesäureester Natriumsalz wobei man eine weisse zähe Paste erhält. Diese wird ohne Zusatz von weiteren Additiven in eine auf 160°C geheizte Formpresse gegeben und 3 Minuten unter Druck belassen. Nach dem Abkühlen kann das fertige Plastisol aus der Form entnommen werden (Ausbeute 100%).

### 3. Anwendungstechnische Eigenschaften des modifizierten PVC

Die Migrationseigenschaften des modifizierten PVC werden durch Extraktionsexperimente mit Hexan als Extraktionsmittel getestet. Man wiegt eine Probe des modifizierten PVC ein und bedeckt sie unter Rühren mit Hexan. Nach unterschiedlichen Zeitabständen wird die Probe entnommen, getrocknet und erneut gewogen. Aus der Differenz der festgestellten und ursprünglichen Masse kann die Menge an migriertem Weichmacher ermittelt werden. Eine typische Extraktionskurve von einer in dieser Erfindung hergestellten PVC-Probe ist in Fig. 1 dargestellt (untere Kurve). Zum Vergleich ist außerdem eine Extraktionskurve eines mit Dioctylphthalat weichgemachten PVC (obere Kurve) dargestellt.

## Patentansprüche

1. Thiolatverbindung der allgemeinen Formel I 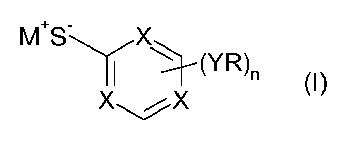 worin
M⁺ für H oder ein Kationäquivalent steht,
n für 1 oder 2 steht,
X unabhängig voneinander für eine Gruppe CH oder N stehen,
Y an ein Ring-C-Atom gebunden ist und unabhängig voneinander für *-CO-O-, *-O-CO-, *-CO-NR'-, *-NR'-CO-, *-SO₂-NR'-, *-NH-CO-O-, *-NH-CO-NR'-, *-O-, *-S- oder *-NR'- steht, wobei * den Anknüpfungspunkt an das Ringsystem beschreibt,
R für einen Polyalkylenoxid-Rest steht, oder, wenn wenigstens ein X für N steht, R auch für (C₆-C₂₂)-Alkyl stehen kann,
R' für Wasserstoff, (C₁-C₄)-Alkyl oder R steht.

2. Thiolatverbindung nach Anspruch 1, der allgemeinen Formel I.a 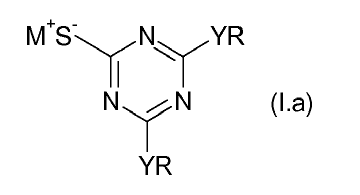 worin M⁺, Y und R die in Anspruch 1 angegebene Bedeutung haben.

3. Thiolatverbindung nach Anspruch 1, der allgemeinen Formel I.b 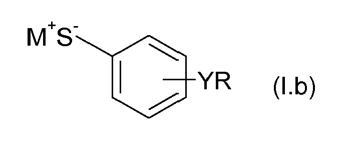 worin M⁺, Y und R die in Anspruch 1 angegebene Bedeutung haben.

4. Thiolatverbindung nach einem der vorhergehenden Ansprüche, worin R die allgemeine Formel aufweist:
-Qₖ-(CₓH₂ₓ-O)ₘ₋R"
worin
k für 0 oder 1 steht,
x für eine ganze Zahl von 2 bis 6 steht,
m für 3 bis 100 steht,
Q für C₂-C₂₀-Alkylen steht und
R" für Wasserstoff oder C₁-C₂₄-Alkyl steht.

5. Thiolatverbindung nach einem der vorhergehenden Ansprüche, aufweisend ein Molekulargewicht von mindestens 600 g/mol.

6. Verwendung einer Thiolatverbindung der Formel I nach einem der vorhergehenden Ansprüche zur Weichmachung von PVC.

7. Modifiziertes PVC, enthaltend Wiederholungseinheiten der Formeln II.a und II.b 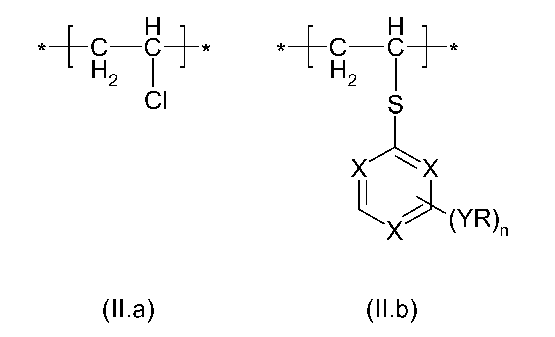 worin n, R, X und Y die in Anspruch 1 angegebene Bedeutung haben.

8. Modifiziertes PVC nach Anspruch 7, worin das molare Verhältnis der Wiederholungseinheiten der Formel II.b zu den Wiederholungseinheiten der Formel II.a 1:200 bis 1:9 beträgt.

9. Verwendung des modifizierten PVC nach Anspruch 7 oder 8 in biomedizinischen Anwendungen, in Kinderspielzeug und in Anwendungen, in denen das modifizierte PVC in Kontakt mit dem menschlichen Organismus oder physiologischen Flüssigkeiten tritt.

10. Verfahren zur Herstellung einer Thiolatverbindung nach Anspruch 3 der allgemeinen Formel I.b, worin Y für *-CO-O-, *-CO-NR'-, *-NH-CO-O- oder *-NH-CO-NR'- steht, indem man
1) ein Disulfid der Formel III.a 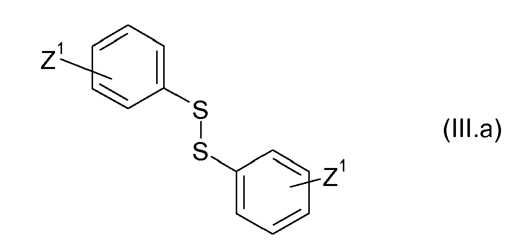 worin Z¹ für CO-Z³ oder NCO steht, wobei Z³ für Halogen oder Acyloxy steht,
mit einer Verbindung der Formel R-Z²H, worin Z² für O oder NR' steht, zum Disulfid der Formel III.b umsetzt, 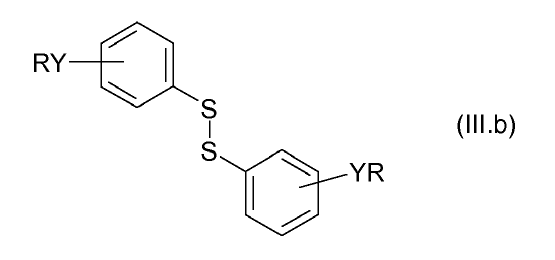 und
2) das Disulfid der Formel III.b in eine Thiolatverbindung der Formel I.b umwandelt.

11. Verfahren zur Herstellung einer Thiolatverbindung nach Anspruch 3 der allgemeinen Formel I.b, worin Y für *-O-CO-, *-NR'-CO-, *-O- oder *-NR'- steht, indem man
1) ein Disulfid der Formel III.c 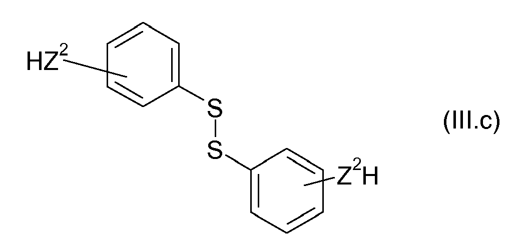 worin Z² für O oder NR' steht, mit einer Verbindung der Formel R-Z⁵, worin Z⁵ für CO-Z³ oder eine Abgangsgruppe steht, wobei Z³ für Halogen oder Acyloxy steht, zum Disulfid der Formel III.d umsetzt, und 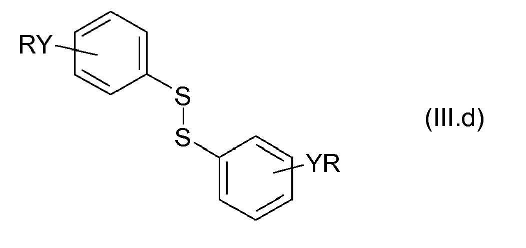
2) das Disulfid der Formel III.d in eine Thiolatverbindung der Formel I.b umwandelt.

12. Verfahren zur Herstellung einer Thiolatverbindung nach Anspruch 3 der allgemeinen Formel I.b, worin Y für *-SO₂-NR'- steht, indem man
1) ein Disulfid der Formel III.e 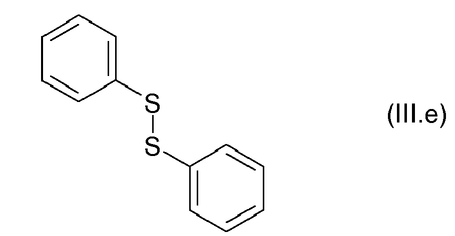 zum Sulfonylchlorid der Formel III.f chlorsulfoniert, 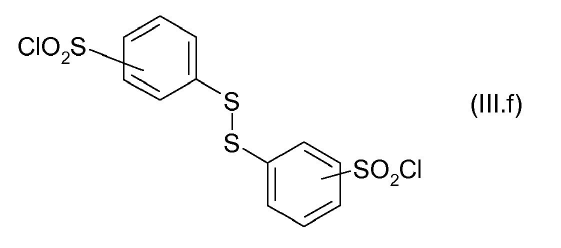
2) das Sulfonylchlorid der Formel III.f mit einer Verbindung der Formel R-Z²H, worin Z² für O oder NR' steht, zum Disulfid der Formel III.g umsetzt, 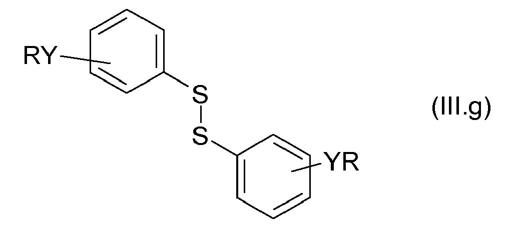
3) das Disulfid der Formel I.e in eine Thiolatverbindung der Formel I.b umwandelt.

13. Verfahren zur Herstelung einer Thiolatverbindung nach Anspruch 3 der allgemeinen Formel I.b, worin Y für *-O- steht, indem man
1) ein Disulfid der Formel III.h 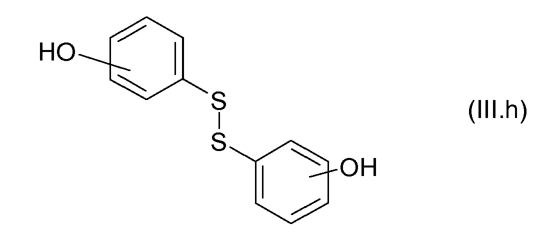 in Gegenwart einer Base mit einem Alkylenoxid der Formel CₓH₂ₓO, worin x für eine ganze Zahl von 2 bis 6 steht, zu einem Disulfid der Formel III.i umsetzt, worin m für 3 bis 100 steht, und 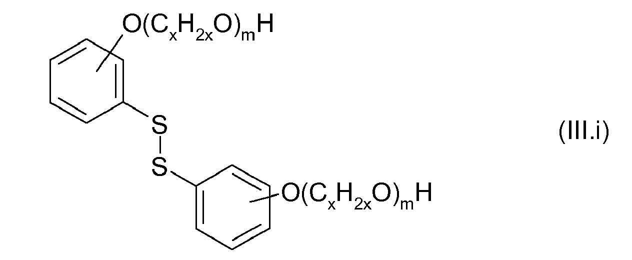
2) das Disulfid der Formel III.i in eine Thiolatverbindung der Formel I.b umwandelt.

14. Verfahren zur Herstellung einer Thiolatverbindung nach Anspruch 3 der Formel I.b, indem man
1) eine Verbindung der Formel IV.c, 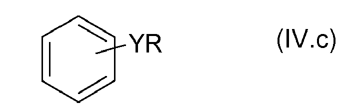 chlorsulfoniert, und
das erhaltene Sulfonylchlorid der Formel IV.d 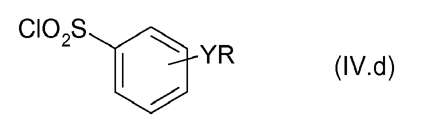 in eine Thiolatverbindung der Formel I.b umwandelt.

15. Verfahren zur Herstellung einer Thiolatverbindung nach Anspruch 2 der Formel I.a, worin Y für *-O-, *-S- oder *-NR'- steht, indem man
1) ein [1,3,5]-Triazin der Formel V.a 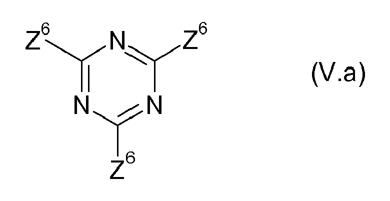 worin Z⁶ für eine Abgangsgruppe steht
mit wenigstens einer Verbindung der Formel R-Z²H, worin Z² für O oder NR' steht, zu einer Verbindung der Formel V.b umsetzt, 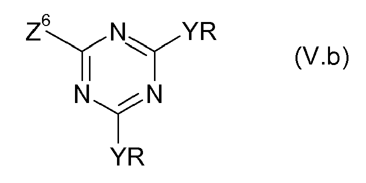
2) die Verbindung der Formel V.b mit Thioharnstoff zu einem Thiuroniumsalz
der Formel V.c umsetzt, und 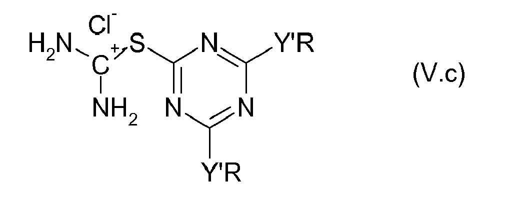
3) das erhaltene Thiouroniumsalz durch Umsetzung mit einer Base in die Thiolatverbindung der Formel I.a umwandelt.
